# EUROPEAN PATENT SPECIFICATION

(11) **EP 1 395 829 B1**
(45) Date of publication and mention of the grant of the patent: **11.07.2007**
(21) Application number: 02730520.0
(22) Date of filing: 14.06.2002
(51) Int. Cl.: G01N 33/574, C12Q 1/68, A61K 39/00, A61K 39/395, C07K 16/30, C07K 14/47, A61P 35/04, C12N 15/11

(54) **CANCER ASSOCIATED PROTEIN**
KREBS-ASSOZIIERTES PROTEIN
PROTEINE ASSOCIEE AU CANCER

(30) Priority: 15.06.2001 GB 0114644
(43) Date of publication of application: 10.03.2004
(73) Proprietor: UCB, S.A., 1070 Bruxelles (BE)
(72) Inventor: TERRETT, Jonathan A., Oxford GlycoSciences (UK)Ltd, Abingdon, Oxfordshire OX14 4RY (GB)
(74) Representative: Thompson, John
(86) International application number: PCT/GB2002/002779
(87) International publication number: WO 2002/103362

(56) References cited:
- WO-A-00/55351
- WO-A-01/98318
- PATENT ABSTRACTS OF JAPAN vol. 1999, no. 11, 30 September 1999 (1999-09-30) & JP 11 146790 A (SUMITOMO PHARMACEUT CO LTD), 2 June 1999 (1999-06-02)
- SUPERTI-FURGA ANDREA ET AL: "Achondrogenesis type IB is caused by mutations in the diastrophic dysplasia sulphate transporter gene." NATURE GENETICS, vol. 12, no. 1, 1996, pages 100-102, XP008020210 ISSN: 1061-4036 cited in the application -& DATABASE SWISS-PROT [Online] ID: DTD_HUMAN, 1 October 1996 (1996-10-01) "SLC26A2 (DTDST, DTD)" Database accession no. P50443 XP002249419
- HASTBACKA JOHANNA ET AL: "Atelosteogenesis type II is caused by mutations in the diastrophic dysplasia sulfate-transporter gene (DTDST): Evidence for a phenotypic series involving three chondrodysplasias." AMERICAN JOURNAL OF HUMAN GENETICS, vol. 58, no. 2, 1996, pages 255-262, XP008020209 ISSN: 0002-9297 cited in the application & DATABASE SWISS-PROT [Online] ID: DTD_HUMAN, 1 October 1996 (1996-10-01) "SLC26A2 (DTDST, DTD)" Database accession no. P50443 XP002249419

### SEQUENCE LISTING

<110> Oxford GlycoSciences (UK) Ltd
   Terrett, Jonathan A
<120> PROTEIN
<130> P0082-W001
<150> GB 0114644.8
   <151> 2001-06-15
<160> 8
<170> Patent In version 3.1
<210> 1
   <211> 739
   <212> PRT
   <213> Homo sapiens
<400> 1
   Met Ser Ser Glu Ser Lys Glu Gln His Asn Val Ser Pro Arg Asp Ser
   1               5                   10                  15
   Ala Glu Gly Asn Asp Ser Tyr Pro Ser Gly Ile His Leu Glu Leu Gln
               20                  25                  30
   Arg Glu Ser Ser Thr Asp Phe Lys Gln Phe Glu Thr Asn Asp Gln Cys
           35                  40                  45
   Arg Pro Tyr His Arg Ile Leu Ile Glu Arg Gln Glu Lys Ser Asp Thr
       50                  55                  60
   Asn Phe Lys Glu Phe Val Ile Lys Lys Leu Gln Lys Asn Cys Gln Cys
   65                  70                  75                  80
   Ser Pro Ala Lys Ala Lys Asn Met Ile Leu Gly Phe Leu Pro Val Leu
                   85                  90                  95
   Gln Trp Leu Pro Lys Tyr Asp Leu Lys Lys Asn Ile Leu Gly Asp Val
               100                 105                 110
   Met Ser Gly Leu Ile Val Gly Ile Leu Leu Val Pro Gln Ser Ile Ala
           115                 120                 125
   Tyr Ser Leu Leu Ala Gly Gln Glu Pro Val Tyr Gly Leu Tyr Thr Ser
       130                 135                 140
   Phe Phe Ala Ser Ile Ile Tyr Phe Leu Leu Gly Thr Ser Arg His Ile
   145                 150                 155                 160
   Ser Val Gly Ile Phe Gly Val Leu Cys Leu Met Ile Gly Glu Thr Val
                   165                 170                 175
   Asp Arg Glu Leu Gln Lys Ala Gly Tyr Asp Asn Ala His Ser Ala Pro
               180                 185                 190
   Ser Leu Gly Met Val Ser Asn Gly Ser Thr Leu Leu Asn His Thr Ser
           195                 200                 205
   Asp Arg Ile Cys Asp Lys Ser Cys Tyr Ala Ile Met Val Gly Ser Thr
       210                 215                 220
   Val Thr Phe Ile Ala Gly Val Tyr Gln Val Ala Met Gly Phe Phe Gln
   225                 230                 235                 240
   Val Gly Phe Val Ser Val Tyr Leu Ser Asp Ala Leu Leu Ser Gly Phe
                   245                 250                 255
   Val Thr Gly Ala Ser Phe Thr Ile Leu Thr Ser Gln Ala Lys Tyr Leu
               260                 265                 270
   Leu Gly Leu Asn Leu Pro Arg Thr Asn Gly Val Gly Ser Leu Ile Thr
           275                 280                 285
   Thr Trp Ile His Val Phe Arg Asn Ile His Lys Thr Asn Leu Cys Asp
       290                 295                 300
   Leu Ile Thr Ser Leu Leu Cys Leu Leu Val Leu Leu Pro Thr Lys Glu
   305                 310                 315                 320
   Leu Asn Glu His Phe Lys Ser Lys Leu Lys Ala Pro Ile Pro Ile Glu
                   325                 330                 335
   Leu Val Val Val Val Ala Ala Thr Leu Ala Ser His Phe Gly Lys Leu
               340                 345                 350
   His Glu Asn Tyr Asn Ser Ser Ile Ala Gly His Ile Pro Thr Gly Phe
   355 360 365
   Met Pro Pro Lys Val Pro Glu Trp Asn Leu Ile Pro Ser Val Ala Val
   370 375 380
   Asp Ala Ile Ala Ile Ser Ile Ile Gly Phe Ala Ile Thr Val Ser Leu
   385 390 395 400
   Ser Glu Met Phe Ala Lys Lys His Gly Tyr Thr Val Lys Ala Asn Gln
   405 410 415
   Glu Met Tyr Ala Ile Gly Phe Cys Asn Ile Ile Pro Ser Phe Phe His
   420 425 430
   Cys Phe Thr Thr Ser Ala Ala Leu Ala Lys Thr Leu Val Lys Glu Ser
   435 440 445
   Thr Gly Cys His Thr Gln Leu Ser Gly Val Val Thr Ala Leu Val Leu
   450 455 460
   Leu Leu Val Leu Leu Val Ile Ala Pro Leu Phe Tyr Ser Leu Gln Lys
   465 470 475 480
   Ser Val Leu Gly Val Ile Thr Ile Val Asn Leu Arg Gly Ala Leu Arg
   485 490 495
   Lys Phe Arg Asp Leu Pro Lys Met Trp Ser Ile Ser Arg Met Asp Thr
   500 505 510
   Val Ile Trp Phe Val Thr Met Leu Ser Ser Ala Leu Leu Ser Thr Glu
   515 520 525
   Ile Gly Leu Leu Val Gly Val Cys Phe Ser Ile Phe Cys Val Ile Leu
   530 535 540
   Arg Thr Gln Lys Pro Lys Ser Ser Leu Leu Gly Leu Val Glu Glu Ser
   545 550 555 560
   Glu Val Phe Glu Ser Val Ser Ala Tyr Lys Asn Leu Gln Thr Lys Pro
   565 570 575
   Gly Ile Lys Ile Phe Arg Phe Val Ala Pro Leu Tyr Tyr Ile Asn Lys
   580 585 590
   Glu Cys Phe Lys Ser Ala Leu Tyr Lys Gln Thr Val Asn Pro Ile Leu
   595 600 605
   Ile Lys Val Ala Trp Lys Lys Ala Ala Lys Arg Lys Ile Lys Glu Lys
   610 615 620
   Val Val Thr Leu Gly Gly Ile Gln Asp Glu Met Ser Val Gln Leu Ser
   625 630 635 640
   His Asp Pro Leu Glu Leu His Thr Ile Val Ile Asp Cys Ser Ala Ile
   645 650 655
   Gln Phe Leu Asn Thr Ala Gly Ile His Thr Leu Lys Glu Val Arg Arg
   660 665 670
   Asp Tyr Glu Ala Ile Gly Ile Gln Val Leu Leu Ala Gln Cys Asn Pro
   675 680 685
   Thr Val Arg Asp Ser Leu Thr Asn Gly Glu Tyr Cys Lys Lys Glu Glu
   690 695 700
   Glu Asn Leu Leu Phe Tyr Ser Val Tyr Glu Ala Met Ala Phe Ala Glu
   705 710 715 720
   Val Ser Lys Asn Gln Lys Gly Val Cys Val Pro Asn Gly Leu Ser Leu
   725 730 735
   Ser Ser Asp
<210> 2
   <211> 2314
   <212> DNA
   <213> Homo sapiens
<400> 2
   aggaagctga accatctatc tccagaaatg tcttcagaaa gtaaagagca acataacgtt 60
   tcacccagag actcagctga aggaaatgac agttatccat ctgggatcca tctggaactt 120
   caaagggaat caagtactga cttcaagcaa tttgagacca atgatcaatg cagaccttat 180
   cataggatcc ttattgagcg tcaagagaaa tcagatacaa acttcaagga gtttgttatt 240
   aaaaagctgc agaagaattg ccagtgcagt ccagccaaag ccaaaaatat gattttaggt 300
   ttccttcctg ttttgcagtg gctcccaaaa tacgacctaa agaaaaacat tttaggggat 360
   gtgatgtcag gcttgattgt gggcatatta ttggtgcccc agtccattgc ttattccctg 420
   ctggctggcc aagaacctgt ctatggtctg tacacatctt tttttgccag catcatttat 480
   tttctcttgg gtacctcccg tcacatctct gtgggcattt ttggagtact gtgccttatg 540
   attggtgaga cagttgaccg agaactacag aaagctggct atgacaatgc ccatagtgct 600
   ccttccttag gaatggtttc aaatgggagc acattattaa atcatacatc agacaggata 660
   tgtgacaaaa gttgctatgc aattatggtt ggcagcactg taacctttat agctggagtt 720
   tatcaggtag cgatgggctt ctttcaagtg ggttttgttt ctgtctacct ctcagatgcc 780
   ttgctgagtg gatttgtcac tggtgcctcc ttcactattc ttacatctca ggccaagtat 840
   cttcttgggc tcaaccttcc tcggactaat ggtgtgggct cactcatcac tacctggata 900
   catgtcttca gaaacatcca taagaccaat ctctgtgatc ttatcaccag ccttttgtgc 960
   cttttggttc ttttgccaac caaagaactc aatgaacact tcaaatccaa gcttaaggca 1020
   ccgattccta ttgaacttgt tgttgttgta gcagccacat tagcctctca ttttggaaaa 1080
   ctacatgaaa attataattc tagtattgct ggacatattc ccactgggtt tatgccaccc 1140
   aaagtaccag aatggaacct aattcctagt gtggctgtag atgcaatagc tatttccatc 1200
   attggttttg ctatcactgt atcactttct gagatgtttg ccaagaaaca tggttacaca 1260
   gtcaaagcaa accaggaaat gtatgccatt ggcttttgta atatcatccc ttccttcttc 1320
   cactgtttta ctactagtgc agctcttgca aagacattgg ttaaagaatc aacaggctgc 1380
   catactcagc tttctggtgt ggtaacagcc ctggttcttt tgttggtcct cctagtaata 1440
   gctcctttgt tctattccct tcaaaaaagt gtccttggtg tgatcacaat tgtaaatcta 1500
   cggggagccc ttcgtaaatt tagggatctt cccaaaatgt ggagtattag tagaatggat 1560
   acagttatct ggtttgttac tatgctgtcc tctgcactgc taagtactga aataggccta 1620
   cttgttgggg tttgtttttc tatattttgt gtcatcctcc gcactcagaa gccaaagagt 1680
   tcactgcttg gcttggtgga agagtctgag gtctttgaat ctgtgtctgc ttacaagaac 1740
   cttcagacta agccaggcat caagattttc cgctttgtag cccctctcta ctacataaac 1800
   aaagaatgct ttaaatctgc tttatacaaa caaactgtca acccaatctt aataaaggtg 1860
   gcttggaaga aggcagcaaa gagaaagatc aaagaaaaag tagtgactct tggtggaatc 1920
   caggatgaaa tgtcagtgca actttcccat gatcccttgg agctgcatac tatagtgatt 1980
   gactgcagtg caattcaatt tttaaataca gcagggatcc acacactgaa agaagttcgc 2040
   agagattatg aagccattgg aatccaggtt ctgctggctc agtgcaatcc cactgtgagg 2100
   gattccctaa ccaacggaga atattgcaaa aaggaagaag aaaaccttct cttctatagt 2160
   gtgtatgaag cgatggcttt tgcagaagta tctaaaaatc agaaaggagt atgtgttccc 2220
   aatggtctga gtcttagtag tgattaattg agaaggtaga tagaagaatg tctagccaat 2280
   aggttaggat ttcaagtgtc caacatttcc cagt 2314
<210> 3
   <211> 22
   <212> DNA
   <213> Artificial Sequence
<220>
   <223> Primer
<400> 3
   aggaagctga accatctatc tc 22
<210> 4
   <211> 22
   <212> DNA
   <213> Artificial Sequence
<220>
   <223> Primer
<400> 4
   actgggaaat gttggacact tg 22
<210> 5
   <211> 22
   <212> DNA
   <213> Artificial Sequence
<220>
   <223> Primer
<400> 5
   ccagtccatt gcttattccc tg 22
<210> 6
   <211> 22
   <212> DNA
   <213> Artificial Sequence
<220>
   <223> Primer
<400> 6
   gttctcggtc aactgtctca cc 22
<210> 7
   <211> 22
   <212> DNA
   <213> Artificial Sequence
<220>
   <223> Primer
<400> 7
   gctggagttt atcaggtagc ga 22
<210> 8
   <211> 22
   <212> DNA
   <213> Artificial Sequence
<220>
   <223> Primer
<400> 8
   ttgcatctac agccacacta gg 22

## Description

The present invention relates to a sulphate transporter protein (DTD) and its involvement in cancer, particularly breast cancer, compositions comprising the protein and antibodies which are immunospecific for the protein. The use of the protein in the diagnosis, screening, treatment and prophylaxis of breast cancer is also provided.

### Breast Cancer

Breast cancer is the most frequently diagnosed non-skin cancer among women in the United States. It is second only to lung cancer in cancer-related deaths. In the UK, breast cancer is by far the commonest cancer in women, with 34,600 new cases in 1998 (Cancer Research Campaign, http://www.crc.org.uk, UK, 2000). Ninety-nine percent of breast cancers occur in women. The risk of developing breast cancer steadily increases with age; the lifetime risk of developing breast cancer is estimated to be 1 in 8 for women in the US. The annual cost of breast cancer treatment in the United States is approximately $10 billion (Fuqua, et. al. 2000, American Association for Cancer Research, www.aacr.org, USA). Breast cancer incidence has been rising over the past five decades, but recently it has reached a plateau. This may reflect a period of earlier detection of breast cancers by mammography. A number of established factors can increase a woman's risk of having the disease. These include older age, history of prior breast cancer, significant radiation exposure, strong family history of breast cancer, upper socioeconomic class, nulliparity, early menarche, late menopause, or age at first pregnancy greater than 30 years. Prolonged use of oral contraceptives earlier in life appears to increase risk slightly. Prolonged postmenopausal oestrogen replacement increases the risk 20 to 40%. It has been speculated that a decrease in the age at menarche, changing birth patterns, or a rise in the use of exogenous estrogens has contributed to the increase in breast cancer incidence (Fuqua, et. al. 2000, American Association for Cancer Research, www.aacr.org, USA).

### Causes of Breast Cancer

Breast cancer is a heterogeneous disease. Although female hormones play a significant role in driving the origin and evolution of many breast tumours, there are a number of other recognised and unknown factors involved. Perturbations in oncogenes identified include amplification of the HER-2 and the epidermal growth factor receptor genes, and over-expression of cyclin D1. Over-expression of these oncogenes has been associated with a significantly poorer prognosis. Similarly, genetic alterations or the loss of tumour suppressor genes, such as the p53 gene, have been well documented in breast cancer and are also associated with a poorer prognosis. Researchers have identified two genes, called BRCA1 and BRCA2, which are predictive of premenopausal familial breast cancer. Genetic risk assessment is now possible, which may enhance the identification of candidates for chemoprevention trials (Fuqua, et. al. 2000, American Association for Cancer Research, www.aacr.org, USA).

### Diagnosis

Early diagnosis of breast cancer is vital to secure the most favourable outcome for treatment. Many countries with advanced healthcare systems have instituted screening programs for breast cancer. This typically takes the form of regular x-ray of the breast (mammography) during the 50-60 year old age interval where greatest benefit for this intervention has been shown. Some authorities have advocated the extension of such programs beyond 60 and to the 40-49 age group. Health authorities in many countries have also promoted the importance of regular breast self-examination by women. Abnormalities detected during these screening procedures and cases presenting as symptomatic would typically be confirmed by aspiration cytology, core needle biopsy with a stereotactic or ultrasound technique for non-palpable lesions, or incisional or excisional biopsy. At the same time other information relevant to treatment options and prognosis, such as oestrogen (ER) and progesterone receptor (PR) status would typically be determined (National Cancer Institute, USA, 2000, Breast Cancer PDQ, www.nci.org).

### Disease Staging and Prognosis

Staging of breast cancer is the key to choosing the optimum treatment for each patient and to select those patients who will fare well with less intensive forms of therapy from those for whom intensive therapy is essential. Currently the process of staging involves lump and axillary lymph node biopsies, combined with extensive histopathology. Patients can be incorrectly staged with consequent over- or under-treatment. There is a need for new markers that can be correlated with disease stage and used to reliably guide treatment decisions. Such new markers would not only benefit patients and health care providers by selecting the optimum treatment, but could provide significant cost and time benefits in the histology lab.

However, some breast tumours become refractory to treatment, as the cancer cells develop resistance to chemotherapy drugs or lose their hormone sensitivity, leading to recurrent or metastatic disease which is often incurable. More recently, attention has focussed on the development of immunological therapies (Green, et al. Cancer Treat. Rev. 26, 269-286 (2000); Davis, I.D. Immunol. Cell Biol. 78, 179-195 (2000); Knuth, et al. Cancer Chemother Pharmacol. 46, S46-51 (2000); Shiku, et al. Cancer Chemother. Pharmacol. 46, S77-82 (2000); Saffran, et al. Cancer Metastasis Rev. 18, 437-449 (1999)), such as cancer vaccines and monoclonal antibodies (mAbs), as a means of initiating and targeting a host immune response against tumour cells. In 1998 the FDA approved the use of Herceptin^{™} (Stebbing, et al. Cancer Treat. Rev. 26, 287-290 (2000); Dillman, et al. Cancer Biother. Radiopharm. 14, 5-10 (1999); Miller, et al. Invest. New Drugs 17, 417-427 (1999)), a mAb that recognises the erbB2/HER2-neu receptor protein, as a treatment for metastatic breast cancer. In combination with chemotherapy, Herceptin^{™} has been shown to prolong the time to disease progression, when compared to patients receiving chemotherapy alone (Baselga, et al. Cancer Res. 58, 2825-2831 (1998)). Herceptin^{™}, however, is only effective in treating the 10-20% of patients whose tumours over-express the erbB2 protein. Thus, the identification of other suitable targets or antigens for immunotherapy of breast cancer has become increasingly important.

An ideal protein target for cancer immunotherapy should have a restricted expression profile in normal tissues and be over-expressed in tumours, such that the immune response will be targeted to tumour cells and not against other organs. In addition, the protein target should be exposed on the cell surface, where it will be accessible to therapeutic agents. Tumour antigens have been identified for a number of cancer types, by using techniques such as differential screening of cDNA (Hubert, et al. Proc. Natl. Acad, Sci. USA 96, 14523-14528 (1999); Lucas, et al. Int. J. Cancer 87, 55-60 (2000)), and the purification of cell-surface proteins that are recognised by tumour-specific antibodies (Catimel, et al. J. Biol. Chem. 271, 25664-25670 (1996)).

### DTD

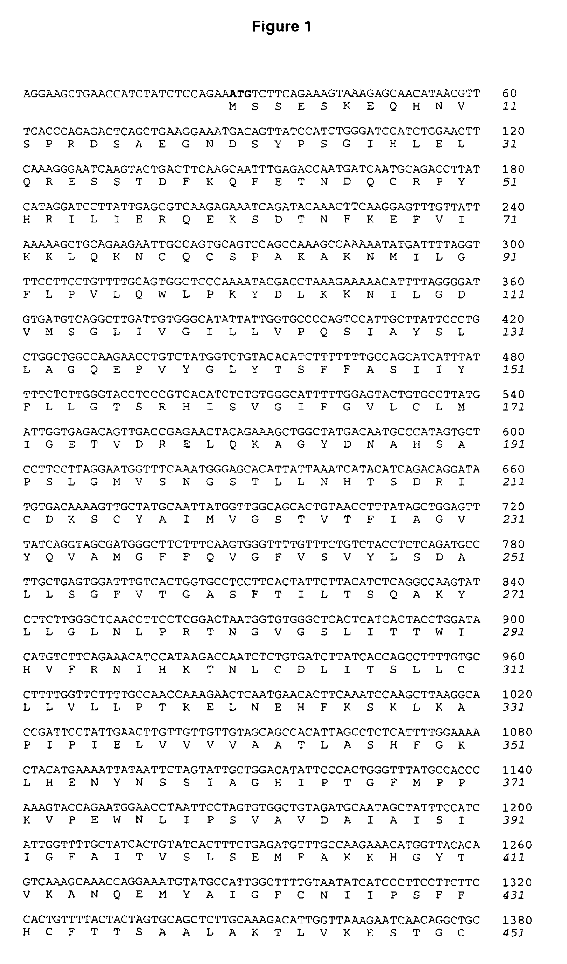

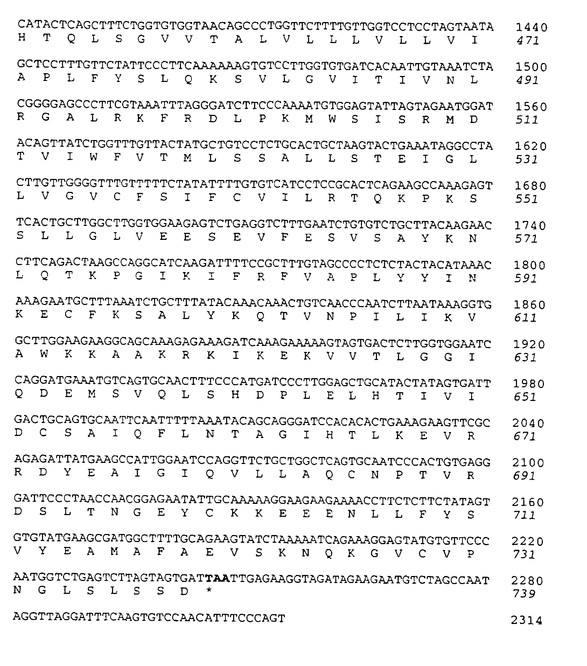

The Diastrophic Dysplasia (DTD) gene was cloned in 1994 (Hastbacka J, et al. Cell 1994, 78(6):1073-87). Its sequence is stored in the Swiss-Prot database (held by the Swiss Institute of Bioinformatics (SIB) and available at http://www.expasy.ch/) under the accession number P50443 and encodes a putative 12-membrane sulfate transporter of 739 amino acids (Figure 1, SEQ ID NO: 1). It is located on chromosome 5q31-q34.

Mutations in DTD are associated with a family of recessively inherited osteochondrodysplasias including Diastrophic Dysplasia (Hastbacka *et al., supra*), Achondrogenesis Type IB (Superti-Furga A, et al. Nat Genet 1996, 12(1):100-2) and Atelosteogenesis Type II (Hastbacka J, et al. Am J Hum Genet 1996, 58(2):255-62). JP11146790 discloses a novel vector for the expression of DTD and the use thereof to screen for agents treating a human bone/cartilage disease, as well as the use of the vector in the preparation of a drug for the treatment of human bone/cartilage diseases.

WO00/55351 discloses 774 colon cancer antigen sequences including one sequence identical to DTD. Further disclosed is the use of antibodies specific for said sequences which may be of use in the treatment and diagnosis of a pathological condition associated with overexpression of said sequences.

The present invention is based on the finding that the protein DTD shows restricted expression to a few tissues, with elevated expression in breast tumours, suggesting that it may be a suitable target for cancer therapy and diagnosis.

Thus, the present invention provides a method of screening for and/or diagnosis of breast cancer in a subject, which method comprises the step of detecting and/or quantifying the amount of a polypeptide in a biological sample obtained from said subject, wherein the polypeptide:
a) comprises or consists of the amino acid sequence shown in Figure 1 (SEQ ID NO: 1);
b) is a derivative having one or more amino acid substitutions, deletions or insertions relative to the amino acid sequence shown in Figure 1 (SEQ ID NO: 1) which retains the activity of DTD; or
c) is a fragment of a polypeptide as defined in a) or b) above, which is at least ten amino acids long which retains the activity of DTD.

In the present application, the term "polypeptides for use in the invention" is used to refer to all polypeptides described in a) to c) above.

The present invention therefore relates to the diagnosis of breast cancer and the use of an antibody which binds specifically to a DTD polypeptid for the preparation of a medicament for the prophylaxis/treatment of breast cancer in a subject which may be a mammal and is preferably a human.

The polypeptides for use in the invention may be provided in isolated or recombinant form, and may be fused to other moieties. In particular, fusions of the polypeptides of the invention with localisation-reporter polypeptides such as the Green Fluorescent Protein (U.S. Patent Nos. 5,625,048, 5,777,079, 6,054,321 and 5,804,387) or the DsRed fluorescent protein (Matz, et al (1999) Nature Biotech. 17:969-973) are specifically contemplated. The polypeptides for use in the invention, may be provided in substantially pure form, that is to say free, to a substantial extent, from other polypeptides. Thus, a polypeptide for use in the present invention may be provided in a composition in which it is the predominant component present (i.e. it is present at a level of at least 50%; preferably at least 75%, at least 80%, at least 85%, at least 90%, or at least 95%; when determined on a weight/weight basis excluding solvents or carriers).

In order to more fully appreciate the present invention, polypeptides within the scope of a)-c) above will now be discussed in greater detail. Polypeptides within the scope of a)

A polypeptide within the scope of a), may consist of the particular amino acid sequence given in Figure 1 (SEQ ID NO: 1) or may have an additional N-terminal and/or an additional C-terminal amino acid sequence relative to the sequence given in Figure 1 (SEQ ID NO: 1).

Additional N-terminal or C-terminal sequences may be provided for various reasons. Techniques for providing such additional sequences are well known in the art.

Additional sequences may be provided in order to alter the characteristics of a particular polypeptide. This can be useful in improving expression or regulation of expression in particular expression systems. For example, an additional sequence may provide some protection against proteolytic cleavage. This has been done for the hormone Somatostatin by fusing it at its N-terminus to part of the β galactosidase enzyme (Itakwa et al., Science 198: 105-63 (1977)).

Additional sequences can also be useful in altering the properties of a polypeptide to aid in identification or purification. For example, a fusion polypeptide may be provided in which a polypeptide is linked to a moiety capable of being isolated by affinity chromatography. The moiety may be an antigen or an epitope and the affinity column may comprise immobilised antibodies or immobilised antibody fragments which bind to said antigen or epitope (desirably with a high degree of specificity). The fusion polypeptide can usually be eluted from the column by addition of an appropriate eluant.

Additional N-terminal or C-terminal sequences may, however, be present simply as a result of a particular technique used to obtain a polypeptide and need not provide any particular advantageous characteristic to the polypeptide. Such polypeptides are within the scope of the present invention.

Whatever additional N-terminal or C-terminal sequence is present, it is preferred that the resultant polypeptide should exhibit the immunological or biological activity of the polypeptide having the amino acid sequence shown in Figure 1 (SEQ ID NO: 1). Polypeptides within the scope of b)

Turning now to the polypeptides defined in b) above, it will be appreciated by the person skilled in the art that these polypeptides are derivatives of the polypeptide given in a) (SEQ ID NO: 1) above, provided that such derivatives preferably exhibit the immunological or biological activity of the polypeptide having the amino acid sequence shown in Figure 1 (SEQ ID NO: 1). As such, it will be appreciated by one skilled in the art that derivatives can include post-translational modifications, for example but without limitation, phosphorylation, glycosylation and farnesylation.

As used herein, the term "derivative" refers to a polypeptide that comprises an amino acid sequence of a parent polypeptide that has been altered by the introduction of amino acid residue substitutions, deletions or additions, and/or amino acid modifications such as but not limited to, phosphorylation and glycosylation. The derivative polypeptide possesses a similar or identical activity to the parent polypeptide.

As used herein, the term "fragment" refers to a polypeptide comprising an amino acid sequence of at least 5 amino acid residues (preferably, at least 10 amino acid residues, at least 15 amino acid residues, at least 20 amino acid residues, at least 25 amino acid residues, at least 30 residues, at least 40 amino acid residues, at least 50 amino acid residues, at least 60 amino residues, at least 70 amino acid residues, at least 80 amino acid residues, at least 90 amino acid residues, at least 100 amino acid residues, at least 200 amino acid residues or at least 300 amino acid residues) of the amino acid sequence of a parent polypeptide.

Alterations in the amino acid sequence of a polypeptide which do not affect the activity of a polypeptide can occur. These include amino acid deletions, insertions and substitutions and can result from alternative splicing and/or the presence of multiple translation start sites and/or stop sites. Polymorphisms may arise as a result of the infidelity of the translation process. Thus changes in amino acid sequence may be tolerated which do not affect the protein's biological or immunological activity.

The skilled person will appreciate that various changes can often be made to the amino acid sequence of a polypeptide which has a biological or particular activity to produce derivatives (sometimes known as variants or "muteins") having at least a proportion of said activity, and preferably having a substantial proportion of said activity. Such derivatives of the polypeptides described in a) (SEQ ID NO: 1) above are within the scope of the present invention and are discussed in greater detail below. They include allelic and non-allelic derivatives.

An example of a derivative of the polypeptide for use in the present invention is a polypeptide as defined in a) (SEQ ID NO: 1) above, apart from the substitution of one or more amino acids with one or more other amino acids provided said derivative retains the activity of DTD. The skilled person is aware that various amino acids have similar properties. One or more such amino acids of a polypeptide can often be substituted by one or more other such amino acids without eliminating a desired activity of that polypeptide.

Thus, the amino acids glycine, alanine, valine, leucine and isoleucine can often be substituted for one another (amino acids having aliphatic side chains). Of these possible substitutions, it is preferred that glycine and alanine are used to substitute for one another (since they have relatively short side chains) and that valine, leucine and isoleucine are used to substitute for one another (since they have larger aliphatic side chains which are hydrophobic).

Other amino acids which can often be substituted for one another include:
- phenylalanine, tyrosine and tryptophan (amino acids having aromatic side chains);
- lysine, arginine and histidine (amino acids having basic side chains);
- aspartate and glutamate (amino acids having acidic side chains);
- asparagine and glutamine (amino acids having amide side chains);
- cysteine and methionine (amino acids having sulphur-containing side-chains); or
- aspartic acid and glutamic acid can substitute for phospho-serine and phospho-threonine, respectively (amino acids with acidic side chains).

Substitutions of this nature are often referred to as "conservative" or "semi-conservative" amino acid substitutions.

Amino acid deletions or insertions may also be made relative to the amino acid sequence given in a) (SEQ ID NO: 1) above. Thus, for example, amino acids which do not have a substantial effect on the biological and/or immunological activity of the polypeptide, or at least which do not eliminate such activity, may be deleted. Such deletions can be advantageous since the overall length and the molecular weight of a polypeptide can be reduced whilst still retaining activity. This can enable the amount of polypeptide required for a particular purpose to be reduced - for example, dosage levels can be reduced.

Amino acid insertions relative to the sequence given in a) (SEQ ID NO: 1) above can also be made. This may be done to alter the properties of a polypeptide for use in the present invention (e.g. to assist in identification, purification or expression). The skilled person will appreciate that various changes can often be made to the amino acid sequence of a polypeptide as explained above in relation to fusion proteins.

Amino acid changes relative to the sequence given in a) (SEQ ID NO: 1) above can be made using any suitable technique e.g. by using site-directed mutagenesis (Hutchinson et al., 1978, J. Biol. Chem. 253:6551).

It should be appreciated that amino acid substitutions or insertions to the polypeptide for use in the present invention can be made using naturally occurring or non-naturally occurring amino acids. Whether or not natural or synthetic amino acids are used, it is preferred that only L- amino acids are present.

Whatever amino acid changes are made (whether by means of substitution, modification, insertion or deletion), preferred polypeptides for use in the present invention have at least 50% sequence identity with a polypeptide as defined in a) above, more preferably the degree of sequence identity is at least 75%, at least 80%, at least 85%. Sequence identities of at least 90% at least 95%, at least 98% or at least 99% are most preferred.

The term identity can be used to describe the similarity between two polypeptide sequences. The degree of amino acid sequence identity can be calculated using a program such as "bestfit" (Smith and Waterman, Advances in Applied Mathematics, 482-489 (1981)) to find the best segment of similarity between any two sequences. The alignment is based on maximising the score achieved using a matrix of amino acid similarities, such as that described by Schwarz and Dayhof (1979) Atlas of Protein Sequence and Structure, Dayhof, M.O., Ed pp 353-358.

A software package well known in the art for carrying out this procedure is the CLUSTAL program. It compares the amino acid sequences of two polypeptides and finds the optimal alignment by inserting spaces in either sequence as appropriate. The amino acid identity or similarity (identity plus conservation of amino acid type) for an optimal alignment can also be calculated using a software package such as BLASTX. This program aligns the largest stretch of similar sequence and assigns a value to the fit. For any one pattern comparison, several regions of similarity may be found, each having a different score. One skilled in the art will appreciate that two polypeptides of different lengths may be compared over the entire length of the longer fragment. Alternatively small regions may be compared. Normally sequences of the same length are compared for a useful comparison to be made.

Where high degrees of sequence identity are present there will be relatively few differences in amino acid sequence. Thus for example they may be less than 20, less than 10, or even less than 5 differences. Polypeptides within the scope of c)

As discussed *supra,* it is often advantageous to reduce the length of a polypeptide, provided the resultant reduced length polypeptide still has a desired activity or can give rise to useful antibodies. Feature c) therefore covers fragments of polypeptides a) (SEQ ID NO: 1) or b) above for use in the present invention.

The skilled person can determine whether or not a particular fragment has activity. Fragments are at least 10 amino acids long, preferred fragments may be at least 20, at least 30, at least 40, at least 50, at least 75 or at least 100 amino acids long.

A polypeptide as defined herein may be useful as antigenic material, and may be used in the production of vaccines for treatment or prophylaxis of breast cancer and/or pancreatic cancer. Such material can be "antigenic" and/or "immunogenic". Generally, "antigenic" is taken to mean that the protein is capable of being used to raise antibodies or indeed is capable of inducing an antibody response in a subject. "Immunogenic" is taken to mean that the protein is capable of eliciting a protective immune response in a subject. Thus, in the latter case, the protein may be capable of not only generating an antibody response but, in addition, non-antibody based immune responses.

It is well known that is possible to screen an antigenic protein or polypeptide to identify epitopic regions, i.e. those regions which are responsible for the protein or polypeptide's antigenicity or immunogenicity. Methods well known to the skilled person can be used to test fragments and/or homologues and/or derivatives for antigenicity. Thus, the fragments for use in the present invention may include one or more such epitopic regions or be sufficiently similar to such regions to retain their antigenic/immunogenic properties. Thus, for fragments for use according to the present invention the degree of identity is perhaps irrelevant, since they may be 100% identical to a particular part of a protein or polypeptide, homologue or derivative as described herein. The key issue may be that the fragment retains the antigenic/immunogenic properties of the protein from which it is derived.

Homologues, derivatives and fragments may possess at least a degree of the antigenicity /immunogenicity of the protein or polypeptide from which they are derived.

The polypeptide for use in the invention can also be used in the production of a composition for the treatment or prophylaxis of cancer, in particular breast cancer, wherein the composition is a vaccine. The vaccine optionally comprises one or more suitable adjuvants. Examples of adjuvants well-known in the art include inorganic gels, such as aluminium hydroxide, and water-in-oil emulsions, such as incomplete Freund's adjuvant. Other useful adjuvants will be well known to the skilled person.

The skilled person will appreciate that for the preparation of one or more polypeptides for use in the present invention, the preferred approach will be based on recombinant DNA techniques.

These nucleic acid molecules encoding the polypeptides may be used in their own right. Thus, a method of screening for and/or diagnosis of cancer, in particular breast cancer in a subject may comprise the step of detecting and/or quantifying the amount of a nucleic acid in a biological sample obtained from said subject, wherein the nucleic acid molecule:
d) comprises or consists of the DNA sequence shown in Figure 1 (SEQ ID NO: 2) or its RNA equivalent;
e) a sequence which codes for an amino acid molecule as defined in a) (SEQ ID NO: 1), b) or c);
f) a sequence which is complementary to the sequences of d) or e);
g) a sequence which codes for the same polypeptide, as the sequences of d) or e); or
h) a sequence which shows substantial identity with any of those of d), e), f) or g).

In the present application, the term "nucleotides for use in the invention" is used to refer to all nucleotides described in d) to h) above.

The term identity can also be used to describe the similarity between two individual DNA sequences. The 'bestfit' program (Smith and Waterman, Advances in applied Mathematics, 482-489 (1981)) is one example of a type of computer software used to find the best segment of similarity between two nucleic acid sequences, whilst the GAP program enables sequences to be aligned along their whole length and finds the optimal alignment by inserting spaces in either sequence as appropriate. It is preferred if sequences which show substantial identity with any of those of d), e) and f) have e.g. at least 50%, at least 75%, at least 80%, at least 85%, at least 90% or at least 95% sequence identity.

These nucleic acid molecules are now discussed in greater detail.

The polypeptides used in the present invention can be coded for by a large variety of nucleic acid molecules, taking into account the well known degeneracy of the genetic code. All of these molecules can be used in the present invention. They can be inserted into vectors and cloned to provide large amounts of DNA or RNA for further study. Suitable vectors may be introduced into host cells to enable the expression of polypeptides used in the present invention using techniques known to the person skilled in the art.

The term 'RNA equivalent' when used above indicates that a given RNA molecule has a sequence which is complementary to that of a given DNA molecule, allowing for the fact that in RNA 'U' replaces 'T' in the genetic code. The nucleic acid molecule may be in isolated, recombinant or chemically synthetic form.

Techniques for cloning, expressing and purifying polypeptides are well known to the skilled person. DNA constructs can readily be generated using methods well known in the art. These techniques are disclosed, for example in J. Sambrook et al, Molecular Cloning 3rd Edition, Cold Spring Harbour Laboratory Press (2000); in Old & Primrose Principles of Gene Manipulation 5th Edition, Blackwell Scientific Publications (1994); and in Stryer, Biochemistry 4th Edition, W H Freeman and Company (1995). Modifications of DNA constructs and the polypeptide expressed such as the addition of promoters, enhancers, signal sequences, leader sequences, translation start and stop signals and DNA stability controlling regions, or the addition of fusion partners may then be facilitated.

Normally the DNA construct will be inserted into a vector, which may be of phage or plasmid origin. Expression of the polypeptide is achieved by the transformation or transfection of the vector into a host cell, which may be of eukaryotic or prokaryotic origin. Such vectors and suitable host cells form further aspects for use in the present invention.

The nucleotide sequences for use in the present invention, including DNA and RNA, and comprising a sequence encoding a polypeptide for use in the invention, may be synthesised using methods known in the art, such as using conventional chemical approaches or polymerase chain reaction (PCR) amplification. The nucleotide sequences for use in the present invention also permit the identification and cloning of the gene encoding a polypeptide for use in the invention from any species, for instance by screening cDNA libraries, genomic libraries or expression libraries.

Knowledge of the nucleic acid structure can be used to raise antibodies and for gene therapy. Techniques for this are well-known by those skilled in the art, as discussed in more detail herein.

By using appropriate expression systems, polypeptides for use in the invention may be expressed in glycosylated or non-glycosylated form. Non-glycosylated forms can be produced by expression in prokaryotic hosts, such as *E. coli.*

Polypeptides comprising N-terminal methionine may be produced using certain expression systems, whilst in others the mature polypeptide will lack this residue.

Preferred techniques for cloning, expressing and purifying a polypeptide for use in the invention are summarised below:

Polypeptides for use in the invention may be prepared under native or denaturing conditions and then subsequently refolded. Baculoviral expression vectors include secretory plasmids (such as pACGP67 from Pharmingen), which may have an epitope tag sequence cloned in frame (e.g. myc, V5 or His) to aid detection and allow for subsequent purification of the protein. Mammalian expression vectors may include pCDNA3 and pSecTag (both Invitrogen), and pREP9 and pCEP4 (invitrogen). *E. coli* systems include the pBad series (His tagged - Invitrogen) or pGex series (Pharmacia).

In addition to nucleic acid molecules coding for polypeptides for use in the present invention, referred to herein as "coding" nucleic acid molecules, the present specification also includes nucleic acid molecules complementary thereto. Thus, for example, both strands of a double stranded nucleic acid molecule are included within the scope of the present invention (whether or not they are associated with one another). Also included are mRNA molecules and complementary DNA Molecules (e.g. cDNA molecules).

The use of nucleic acid molecules which can hybridise to any of the nucleic acid molecules discussed above is also covered by the present specification. Such nucleic acid molecules are referred to herein as "hybridising" nucleic acid molecules. Hybridising nucleic acid molecules can be useful as probes or primers, for example.

Desirably such hybridising molecules are at least 10 nucleotides in length and preferably are at least 25 or at least 50 nucleotides in length. The hybridising nucleic acid molecules preferably hybridise to nucleic acids within the scope of d), e), f), g) or h) above specifically.

Desirably the hybridising molecules will hybridise to such molecules under stringent hybridisation conditions. One example of stringent hybridisation conditions is where attempted hybridisation is carried out at a temperature of from about 35°C to about 65°C using a salt solution that is about 0.9 molar. However, the skilled person will be able to vary such conditions as appropriate in order to take into account variables such as probe length, base composition, type of ions present, etc. For a high degree of selectivity, relatively stringent conditions are used to form the duplexes, such as low salt or high temperature conditions. As used herein, "highly stringent conditions" means hybridisation to filter-bound DNA in 0.5 M NaHPO₄, 7% sodium dodecyl sulphate (SDS), 1 mM EDTA at 65°C, and washing in 0.1xSSC/0.1% SDS at 68 °C (Ausubel F.M. et al., eds., 1989, Current Protocols in Molecular Biology, Vol. I, Green Publishing Associates, Inc., and John Wiley & Sons, Inc., New York, at p. 2.10.3). For some applications, less stringent conditions for duplex formation are required. As used herein "moderately stringent conditions" refers to washing in 0.2xSSC/0.1% SDS at 42°C (Ausubel *et al.,* 1989, *supra*). Hybridisation conditions can also be rendered more stringent by the addition of increasing amounts of formamide, to destabilise the hybrid duplex. Thus, particular hybridisation conditions can be readily manipulated, and will generally be chosen depending on the desired results. In general, convenient hybridisation temperatures in the presence of 50% formamide are: 42°C for a probe which is 95 to 100% identical to the fragment of a gene encoding a polypeptide as defined herein, 37°C for 90 to 95% identity and 32°C for 70 to 90% identity.

In the preparation of genomic libraries, DNA fragments are generated, some of which will encode parts or the whole of a polypeptide as defined herein. The DNA may be cleaved at specific sites using various restriction enzymes. Alternatively, one may use DNAse in the presence of manganese to fragment the DNA, or the DNA can be physically sheared, as for example, by sonication. The DNA fragments can then be separated according to size by standard techniques, including but not limited to agarose and polyacrylamide gel electrophoresis, column chromatography and sucrose gradient centrifugation. The DNA fragments can then be inserted into suitable vectors, including but not limited to plasmids, cosmids, bacteriophages lambda or T₄, and yeast artificial chromosomes (YACs). (See, for example, Sambrook et al., 1989, Molecular Cloning, A Laboratory Manual, ID Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II; Ausubel F.M. et al., eds., 1989, Current Protocols in Molecular Biology, Vol. I, Green Publishing Associates, Inc., and John Wiley & sons, Inc., New York). The genomic library may be screened by nucleic acid hybridisation to labelled probe (Benton & Davis, 1977, Science 196:180; Grunstein & Hogness, 1975, Proc. Natl. Acad. Sci. U.S.A. 72:3961).

Manipulation of the DNA encoding a polypeptide is a particularly powerful technique for both modifying proteins and for generating large quantities of protein for purification purposes. This may involve the use of PCR techniques to amplify a desired nucleic acid sequence. Thus the sequence data provided herein can be used to design primers for use in PCR so that a desired sequence can be targeted and then amplified to a high degree.

Typically, primers will be at least five nucleotides long and will generally be at least ten nucleotides long (e.g. fifteen to twenty-five nucleotides long). In some cases, primers of at least thirty or at least thirty-five nucleotides in length may be used.

As a further alternative chemical synthesis may be used. This may be automated. Relatively short sequences may be chemically synthesised and ligated together to provide a longer sequence.

In addition to being used as primers and/or probes, hybridising nucleic acid molecules for use in the specification be used as anti-sense molecules to alter the expression of polypeptides for use in the specification by binding to complementary nucleic acid molecules. This technique can be used in anti-sense therapy. As used herein, an "antisense" nucleic acid refers to a nucleic acid capable of hybridising by virtue of some sequence complementarity to a portion of an RNA (preferably mRNA) encoding a polypeptide for use in the invention. The antisense nucleic acid may be complementary to a coding and/or non-coding region of a mRNA encoding such a polypeptide. Such antisense nucleic acids have utility as compounds that inhibit expression, and can be used in the treatment or prevention of cancer, in particular breast cancer.

A hybridising nucleic acid molecule for use in the invention may have a high degree of sequence identity along its length with a nucleic acid molecule within the scope of d)-h) above (e.g. at least 50%, at least 75%, at least 80%, at least 85%, at least 90% at least 95%, at least 98% or at least 99% sequence identity). As will be appreciated by the skilled person, the higher the sequence identity a given single stranded nucleic acid molecule has with another nucleic acid molecule, the greater the likelihood that it will hybridise to a nucleic acid molecule which is complementary to that other nucleic acid molecule under appropriate conditions.

Unless the context indicates otherwise, nucleic acid molecules for use in the present specification may have one or more of the following characteristics:
1) they may be DNA or RNA;
2) they may be single or double stranded;
3) they may be provided in recombinant form, e.g. covalently linked to a 5' and/or a 3' flanking sequence to provide a molecule which does not occur in nature;
4) they may be provided without 5' and/or 3' flanking sequences which normally occur in nature;
5) they may be provided in substantially pure form. Thus they may be provided in a form which is substantially free from contaminating proteins and/or from other nucleic acids; and
6) they may be provided with introns or without introns (e.g. as cDNA).

If desired, a gene encoding a polypeptide for use in the invention, a related gene, or related nucleic acid sequences or subsequences, including complementary sequences, can also be used in hybridisation assays. A nucleotide encoding a polypeptide for use in the invention, or subsequences thereof comprising at least 8 nucleotides, can be used as a hybridisation probe. Hybridisation assays can be used for detection, prognosis, diagnosis, or monitoring of conditions, disorders, or disease states, associated with aberrant expression of genes encoding a polypeptide for use in the invention, or for differential diagnosis of patients with signs or symptoms suggestive of cancer, in particular breast cancer. In particular, such a hybridisation assay can be carried out by a method comprising contacting a patient sample containing nucleic acid with a nucleic acid probe capable of hybridising to a DNA or RNA that encodes a polypeptide as defined herein, under conditions such that hybridisation can occur, and detecting or measuring any resulting hybridisation. Nucleotides can be used for therapy of patients having cancer, in particular breast cancer, as described below.

A convenient means for detecting/quantifying the polypeptides for use in the present invention involves the use of antibodies. Thus, the polypeptides for use in the invention also find use in raising antibodies.

Thus, in a further aspect, the present invention provides the use of an antibody which binds to at least one polypeptide for use in the invention for screening for and/or diagnosis of breast cancer in a subject. The antibody is used for detecting and/or quantifying the amount of a polypeptide for use in the invention in a biological sample obtained from said subject.

In one embodiment, binding of antibody in tissue sections can be used to detect aberrant polypeptide localisation or an aberrant level of polypeptide. In a specific embodiment, an antibody to a polypeptide for use in the invention can be used to assay a patient tissue (e.g., a breast biopsy) for the level of the polypeptide where an aberrant level of polypeptide is indicative of cancer in particular breast cancer. As used herein, an "aberrant level" means a level that is increased compared with the level in a subject free from cancer or a reference level. If desired, the comparison can be performed with a matched sample from the same subject, taken from a portion of the body not affected by cancer.

Suitable immunoassays include, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

In another aspect, the present specification provides a method for the prophylaxis and/or treatment of cancer, particularly breast cancer in a subject, which comprises administering to said subject a therapeutically effective amount of an antibody which binds to at least one polypeptide for use in the invention.

In a further aspect, the present invention provides the use of an antibody which specifically binds to at least one polypeptide for use in the invention in the preparation of a composition for use in the prophylaxis and/or treatment of cancer.

Antibodies bind specifically to polypeptides for use in the present invention so that they can be used to purify and/or inhibit the activity of such polypeptides,

Thus, the polypeptide for use in the specification, may be used as an immunogen to generate antibodies which immunospecifically bind such an immunogen. Antibodies of the invention include, but are not limited to polyclonal, monoclonal, bispecific, humanised or chimeric antibodies, single chain antibodies, Fab fragments and F(ab') fragments, fragments produced by a Fab expression library, anti-idiotypic (anti-Id) antibodies, and epitope-binding fragments of any of the above. The term "antibody" as used herein refers to immunoglobulin molecules and immunologically-active portions of immunoglobulin molecules, i.e., molecules that contain an antigen binding site that specifically binds an antigen. The immunoglobulin molecules of the invention can be of any class (e.g., IgG, IgE, IgM, IgD and IgA) or subclass of immunoglobulin molecule.

In the production of antibodies, screening for the desired antibody can be accomplished by techniques known in the art, e.g. ELISA (enzyme-linked immunosorbent assay). For example, to select antibodies, which recognise a specific domain of a polypeptide used in the invention, one may assay generated hybridomas for a product which binds to a polypeptide fragment containing such domain. For selection of an antibody that specifically binds a first polypeptide homologue but which does not specifically bind to (or binds less avidly to) a second polypeptide homologue, one can select on the basis of positive binding to the first polypeptide homologue and a lack of binding to (or reduced binding to) the second polypeptide homologue.

For preparation of monoclonal antibodies (mAbs) directed toward a polypeptide for use in the invention, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs used in the invention may be cultivated *in vitro* or *in vivo.* In an additional embodiment of the invention, monoclonal antibodies can be produced in germ-free animals utilising known technology (PCT/US90/02545).

The mAbs include but are not limited to human mAbs and chimeric mAbs (e.g., human-mouse chimeras). A chimeric antibody is a molecule in which different portions are derived from different animal species, such as those having a human immunoglobulin constant region and a variable region derived from a murine mAb. (See, e.g., U.S. Patent No. 4,816,567; and U.S. Patent No. 4,816397). Humanised antibodies are antibody molecules from non-human species having one or more complementarity determining regions (CDRs) from the non-human species and a framework region from a human immunoglobulin molecule. (See, e.g., U.S. Patent No. 5,585,089).

Chimeric and humanised mAbs can be produced by recombinant DNA techniques known in the art, for example using methods described in WO 87/02671; EP-A-184,187; EP-A-171,496; EP-A-173,494; WO 86/01533; U.S. Patent No. 4,816,567; EP-A-125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are particularly desirable for therapeutic treatment of human patients. Such antibodies can be produced using transgenic mice which are incapable of expressing endogenous immunoglobulin heavy and light chain genes, but which can express human heavy and light chain genes. The transgenic mice are immunised in the normal fashion with a selected antigen, e.g., all or a portion of a polypeptide for use in the invention. mAbs directed against the antigen can be obtained using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM, IgD and IgE antibodies. For an overview of this technology for producing human antibodies, see Lonberg and Huszar (1995), Int. Rev. Immunol. 13:65-93. For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806.

Completely human antibodies, which recognise a selected epitope can be generated using a technique referred to as "guided selection". In this approach a selected non-human monoclonal antibody, e.g., a mouse antibody, is used to guide the selection of a completely human antibody recognising the same epitope. (Jespers et al. (1994) Bio/technology 12:899-903).

The antibodies used in the present invention can also be generated using various phage display methods known in the art. In phage display methods, functional antibody domains are displayed on the surface of phage particles which carry the polynucleotide sequences encoding them. In particular, such phage can be utilised to display antigen binding domains expressed from a repertoire or combinatorial antibody library (e.g., human or murine). Phage expressing an antigen binding domain that binds the antigen of interest can be selected or identified with antigen, e.g., using labelled antigen or antigen bound or captured to a solid surface or bead. Phage used in these methods are typically filamentous phage including fd and M13 binding domains expressed from phage with Fab, Fv or disulphide stabilised Fv antibody domains recombinantly fused to either the phage gene III or gene VIII protein. Phage display methods that can be used to make the antibodies used in the present invention include those disclosed in Brinkman et al., J. Immunol. Methods 182: 41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994);. PCT/GB91/01 134; WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in WO 92/22324; Mullinax et al., BioTechniques 12(6):864-869 (1992); and Sawai et al., AJRI 34:26-34 (1995); and Better et al, Science 240:1041-1043 (1988).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details for generating bispecific antibodies see, for example, Suresh et al., Methods in Enzymology, 1986, 121:210.

The invention provides for the use of functionally-active fragments, derivatives or analogues of the anti-polypeptide immunoglobulin molecules. "Functionally-active" means that the fragment, derivative or analogue is able to elicit anti-anti-idiotype antibodies (i.e., tertiary antibodies) that recognise the same antigen that is recognised by the antibody from which the fragment, derivative or analogue is derived. Specifically, in a preferred embodiment, the antigenicity of the idiotype of the immunoglobulin molecule may be enhanced by deletion of framework and CDR sequences that are C-terminal to the CDR sequence that specifically recognises the antigen. To determine which CDR sequences bind the antigen, synthetic peptides containing the CDR sequences can be used in binding assays with the antigen by any binding assay method known in the art.

The present invention provides the use of antibody fragments such as, but not limited to, F(ab')2 fragments and Fab fragments. Antibody fragments which recognise specific epitopes may be generated by known techniques. F(ab')2 fragments consist of the variable region, the light chain constant region and the CH1 domain of the heavy chain and are generated by pepsin digestion of the antibody molecule. Fab fragments are generated by reducing the disulphide bridges of the F(ab')2 fragments. The invention also provides heavy chain and light chain dimers of the antibodies of the invention, or any minimal fragment thereof such as Fvs or single chain antibodies (SCAs) (e.g., as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

The present specification provides fusion polypeptides of the immunoglobulins of the invention (or functionally active fragments thereof), for example in which the immunoglobulin is fused via a covalent bond (e.g., a peptide bond), at either the N-terminus or the C-terminus to an amino acid sequence of another polypeptide (or portion thereof, preferably at least 10, 20 or 50 amino acid portion of the polypeptide) that is not the immunoglobulin. Preferably the immunoglobulin, or fragment thereof, is covalently linked to the other polypeptide at the N-terminus of the constant domain. As stated above, such fusion polypeptides may facilitate purification, increase half-life *in vivo,* and enhance the delivery of an antigen across an epithelial barrier to the immune system.

The immunoglobulins used in the invention include analogues and derivatives that are either modified, i.e., by the covalent attachment of any type of molecule as long as such covalent attachment that does not impair immunospecific binding. For example, but not by way of limitation, the derivatives and analogues of the immunoglobulins include those that have been further modified, e.g., by glycosylation, acetylation, pegylation, phosphylation, amidation, derivatisation by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analogue or derivative may contain one or more non-classical amino acids.

The foregoing antibodies can be used in methods known in the art relating to the localisation and activity of the polypeptides used in the invention, e.g., for imaging or radioimaging these proteins, measuring levels thereof in appropriate physiological samples, in diagnostic methods, etc. and for radiotherapy.

The antibodies of the specification can be produced by any method known in the art for the synthesis of antibodies, in particular, by chemical synthesis or by recombinant expression, and are preferably produced by recombinant expression technique.

Recombinant expression of antibodies, or fragments, derivatives or analogues thereof, requires construction of a nucleic acid that encodes the antibody. If the nucleotide sequence of the antibody is known, a nucleic acid encoding the antibody may be assembled from chemically synthesised oligonucleotides (e.g., as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g., an antibody cDNA library, or cDNA library generated from any tissue or cells expressing the antibody) by PCR amplification using synthetic primers hybridisable to the 3' and 5' ends of the sequence or by cloning using an oligonucleotide probe specific for the particular gene sequence.

If an antibody molecule that specifically recognises a particular antigen is not available (or a source for a cDNA library for cloning a nucleic acid encoding such an antibody), antibodies specific for a particular antigen may be generated by any method known in the art, for example, by immunising an animal, such as a rabbit, to generate polyclonal antibodies or, more preferably, by generating monoclonal antibodies. Alternatively, a clone encoding at least the Fab portion of the antibody may be obtained by screening Fab expression libraries (e.g., as described in Huse et al., 1989, Science 246:1275-1281) for clones of Fab fragments that bind the specific antigen or by screening antibody libraries (See, e.g., Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, e.g., WO 86/05807; WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulphide bond with an amino acid residue that does not contain a sulphydryl group. Such modifications can be carried out by any method known in the art for the introduction of specific mutations or deletions in a nucleotide sequence, for example, but not limited to, chemical mutagenesis, *in vitro* site directed mutagenesis (Hutchinson et al., 1978, J. BioL Chem. 253:6551**),** PCR based methods, etc.

In addition, techniques developed for the production of "chimeric antibodies" (Morrison et al., 1984, PNAS. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, e.g., humanised antibodies.

Once a nucleic acid encoding an antibody molecule has been obtained, the vector for the production of the antibody molecule may be produced by recombinant DNA technology using techniques well known in the art. Thus, methods for preparing the polypeptides for use in the invention by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2d Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *E. coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; Cockett et al., 1990, BioTechnology 8:2).

A variety of host-expression vector systems may be utilised to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ.* These include but are not limited to micro-organisms such as bacteria (e.g., *E. coli, B. subtilis*) transformed with recombinant bacteriophage DNA, plasmid DNA or cosmid DNA expression vectors containing antibody coding sequences; yeast (e.g., *Saccharomyces, Pichia*) transformed with recombinant yeast expression vectors containing antibody coding sequences; insect cell systems infected with recombinant virus expression vectors (e.g., baculovirus) containing the antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, HEK 293, 3T3 cells) harbouring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the antibody molecule being expressed. For example, when a large quantity of such a polypeptide is to be produced, for the generation of pharmaceutical compositions comprising an antibody molecule, vectors which direct the expression of high levels of fusion polypeptide products that are readily purified may be desirable. Such vectors include, but are not limited, to the *E. coli* expression vector pUR278 (Ruther et al., 1983, EMBO J. 2:1791), in which the antibody coding sequence may be ligated individually into the vector in frame with the lac Z coding region so that a fusion polypeptide is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion polypeptides with glutathione S-transferase (GST). In general, such fusion polypeptides are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example, the polyhedrin gene) of the virus and placed under control of an AcNPV promoter (for example, the polyhedrin promoter). In mammalian host cells, a number of viral-based expression systems (e.g., an adenovirus expression system) may be utilised.

As discussed above, a host cell strain may be chosen which modulates the expression of the inserted sequences, or modifies and processes the gene product in the specific fashion desired. Such modifications (e.g., glycosylation) and processing (e.g., cleavage) of polypeptide products may be important for the activity of the polypeptide.

For long-term, high-yield production of recombinant antibodies, stable expression is preferred. For example, cells lines that stably express an antibody of interest can be produced by transfecting the cells with an expression vector comprising the nucleotide sequence of the antibody and the nucleotide sequence of a selectable (e.g., neomycin or hygromycin), and selecting for expression of the selectable marker. Such engineered cell lines may be particularly useful in screening and evaluation of agents that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors for use within the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, PNAS 77:2197). The coding sequences for the heavy and light chains may comprise cDNA or genomic DNA.

Once the antibody molecule used in the invention has been recombinantly expressed, it may be purified by any method known in the art for purification of an antibody molecule, for example, by chromatography (e.g., ion exchange chromatography, affinity chromatography such as with protein A or specific antigen, and sizing column chromatography), centrifugation, differential solubility, or by any other standard technique for the purification of polypeptides.

Alternatively, any fusion polypeptide may be readily purified by utilising an antibody specific for the fusion polypeptide being expressed. For example, a system described by Janknecht *et al.* allows for the ready purification of non-denatured fusion polypeptides expressed in human cell lines (Janknecht *et al.,* 1991, PNAS **88**:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

In a preferred embodiment, antibodies used in the invention or fragments thereof are conjugated to a diagnostic or therapeutic moiety. The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In, and ⁹⁹Tc.

Antibodies used in the invention or fragments thereof can be conjugated to a therapeutic agent or drug moiety to modify a given biological response. The therapeutic agent or drug moiety is not to be construed as limited to classical chemical therapeutic agents. For example, the drug moiety may be a polypeptide possessing a desired biological activity. Such polypepties may include, for example, a toxin such as abrin, ricin A, pseudomonas exotoxin, or diphtheria toxin; a polypeptides such as tumor necrosis factor, α-interferon, β-interferon, nerve growth factor, platelet derived growth factor, tissue plasminogen activator, a thrombotic agent or an anti-angiogenic agent, e.g., angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moieties to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84 : Biological And Clinical Applications; Pinchera et al. (eds.), pp. 475-506 (1985); "Analysis, Results, And Future Prospective Of The Therapeutic Use Of Radiolabelled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described in U.S. 4,676,980.

An antibody with or without a therapeutic moiety conjugated to it can be used as a therapeutic that is administered alone or in combination with cytotoxic factor(s) and/or cytokine(s).

As used herein "active agent" refers to antibodies against the polypeptides of use in the invention which modulate the expression of and/or activity of the polypeptides for use in the invention.

As discussed herein, active agents of the invention find use in the treatment or prophylaxis of breast cancer.

### Examples

The invention will now be described with reference to the following examples, which should not in any way be construed as limiting the scope of the present invention. The examples refer to the figures in which:
**Figure 1:** shows the amino acid sequence (SEQ ID NO: 1) and nucleic acid sequence (SEQ ID NO: 2) of DTD, the sequence is stored in the Swiss-Prot database (held by the Swiss Institute of Bioinformatics (SIB) and available at http://www.expasy.ch/) under the accession number P50443.

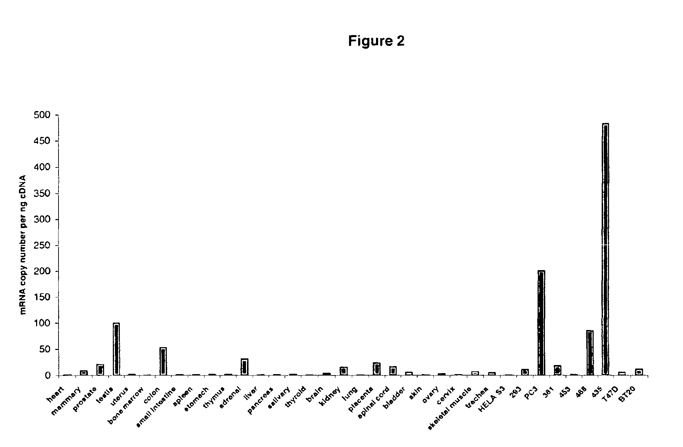

**Figure 2:** shows distribution of DTD mRNA in normal human tissues and a variety of human tumour-derived cell lines. Expression was quantified by real time RT-PCR and mRNA levels are expressed as the number of copies ng⁻¹ cDNA.

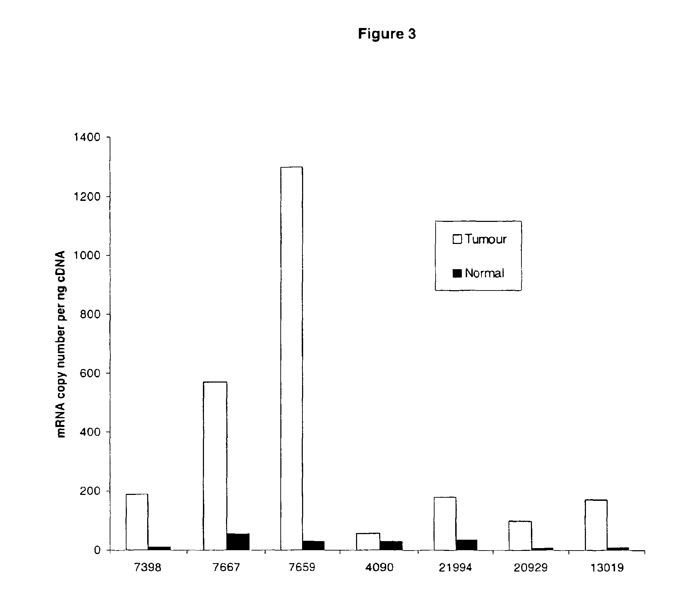

**Figure 3:** shows the expression of DTD mRNA in patient matched adjacent normal and tumour breast tissues. Levels of DTD mRNA in matched normal and tumour tissues from seven breast cancer patients were measured by real time RT-PCR. mRNA levels are expressed as the number of copies ng⁻¹ cDNA.

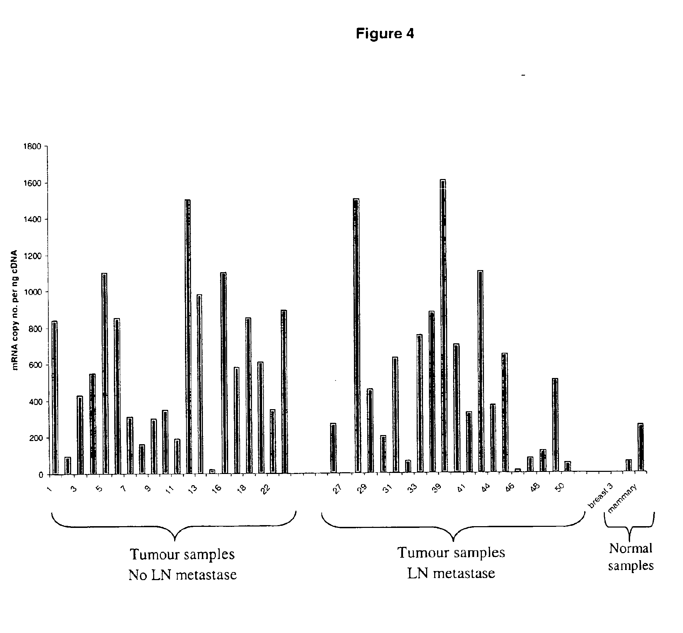

**Figure 4:** shows the expression of DTD in normal and tumour breast tissues. Samples 1-25 are tumour samples not involving metastasis to the lymph nodes. Samples 26-50 are tumour samples involving metastases to variable numbers of lymph nodes. The final 3 samples are from normal breast tissue (reduction mammoplasties). mRNA levels are expressed as the number of copies ng⁻¹ cDNA. There is a statistically significant difference between all tumour samples and normal samples (T-test, p < 0.05).

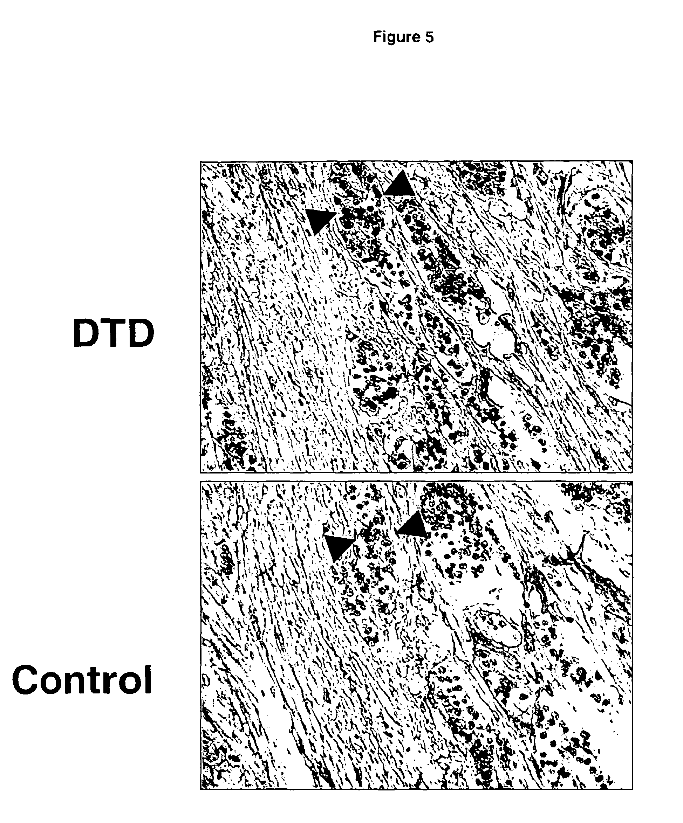

**Figure 5:** *in situ* RT PCR analysis of DTD mRNA expression in sections of invasive ductal breast carcinoma tissue (upper panel), and consecutive negative control section in which the DTD primers have been replaced with primers to a control gene (Prostate Specific Antigen) (lower panel). Note the high DTD expression (dark staining) in a portion of epithelial hyperplasia (typical of breast carcinoma) flanked with two arrowheads in each panel.

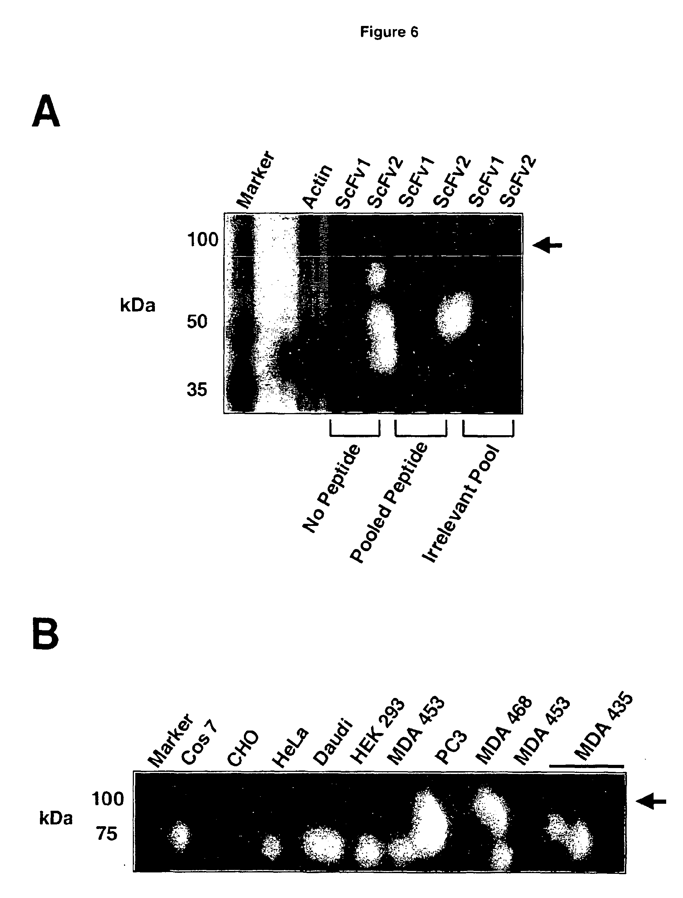

**Figure 6:** western blot analysis of endogenous DTD protein in multiple cell lines using peptide specific single chain Fv (ScFv) antibodies. Two ScFv antibodies that bind denatured DTD were isolated by panning phage against beads conjugated to DTD-derived peptide pools. A, Specific binding of both anti-DTD ScFv's (1 and 2) is demonstrated by the finding that binding to denatured DTD is inhibited by pre-incubation with the pooled peptides used for phage selection. B, Expression of DTD protein in multiple cell lines using anti-DTD ScFvl.

### Example 1: Cloning of DTD

The gene for DTD has been localised to chromosome 5q31-q34 (Hastbacka J, et al. Genomics 1991;11:968-73). A Blast search (http://www.ncbi.nlm.nih.gov/BLAST/) with the DTD gene against High-Throughput Genome Sequences (HTGS) returns the GenBank clone: AC011406. The DTD sequence was amplified using PCR from a colon cDNA library using the following primer pair:
Sense: 5' aggaagctgaaccatctatctc 3' (SEQ ID NO: 3)
Antisense: 5' actgggaaatgttggacacttg 3' (SEQ ID NO: 4)

Alignment of the nucleotide sequence from Figure 1 (SEQ ID NO: 2) and part of the genomic clone AC011406 demonstrated the presence of 2 exons, with the boundary between each exon being around bp724-bp729 in the sequence shown in Figure 1 (SEQ ID NO: 2). Dotlet, accessible at http://www.isrec.isb-sib.ch/java/dotlet/Dotlet.html, was used to align the two sequences.

### Example 2: Expression of DTD mRNA in human tissues and cell lines

Real time quantitative RT-PCR was used (Heid, C.A., Stevens, J., Livak, K.J. & Williams, P.M. Genome Res. 6, 986-994 (1996); Morrison, T.B., et al. Biotechniques 24, 954-958 (1998)) to analyse the distribution of DTD mRNA in normal human tissues and tumour-derived cell lines (Figure 2).

### Quantification of DTD mRNA by Real Time RT -PCR

Real time RT-PCR was used to quantitatively measure DTD expression in cDNA samples derived from: normal human tissues (Clontech), tumour-derived cell lines, breast carcinoma tissues removed during surgery, and normal breast tissue removed during breast reduction mammoplasty. Ethical approval for the normal and tumour breast samples was obtained at surgery (University of Oxford, UK). The primers used for PCR were as follows:
Sense: 5' ccagtccattgcttattccctg 3' (SEQ ID NO: 5)
Antisense: 5' gttctcggtcaactgtctcacc 3' (SEQ ID NO: 6)

Reactions containing 10ng cDNA, SYBR green sequence detection reagents (PE Biosystems) and sense and antisense primers were assayed on an ABI7700 sequence detection system (PE Biosystems). The PCR conditions were 1 cycle at 50°C for 2 min, 1 cycle at 95°C for 10 min, and 40 cycles of 95°C for 15s, 65°C for 1 min. The accumulation of PCR product was measured in real time as the increase in SYBR green fluorescence, and the data were analysed using the Sequence Detector program v1.6.3 (PE Biosystems). Standard curves relating initial template copy number to fluorescence and amplification cycle were generated using the amplified PCR product as a template, and were used to calculate DTD copy number per ng cDNA in each sample.

Figure 2 demonstrates that the distribution of DTD mRNA expression in normal tissues was restricted to testis, colon and adrenal gland, with only low levels of DTD message detected in other normal tissues, including mammary gland. In contrast DTD mRNA was elevated in the MDA-MB-435 breast tumour-derived cell line and to a lesser degree the MDA-MB-468 breast tumour and PC3 prostate tumour cell lines (Figure 2).

Given the finding that DTD mRNA was elevated in the MDA-MB-435 breast cancer derived cell line, DTD mRNA expression was compared in seven patient matched adjacent normal and tumour tissue samples (Figure 3). DTD expression was higher in all the breast tumour samples, relative to their matched normal tissues, with five of the samples showing at least 8-fold greater expression. To further examine the association of DTD mRNA expression with breast tumour tissues we extended the quantification of DTD mRNA levels to a further 4.0 tumour samples, 20 of which were derived from patients with lymph node metastasis and 20 from patients with no metastasis (Figure 4). DTD mRNA expression was elevated in the majority of the breast carcinoma samples, relative to the normal breast tissue controls, however, there was no significant association of expression with lymph node metastasis versus non-lymph node metastasis (Figure 4).

### Example 3: In situ RT-PCR

To further illustrate the involvement of DTD in breast cancer, in situ RT-PCR analysis of DTD expression has been carried out on sections of invasive ductal breast carcinoma tissues.

Formalin fixed, paraffin embedded breast tissues from patients with ductal carcinoma was sectioned (5 microns thick) onto glass slides (provided by Human Research Tissue Bank, Department of Cellular Pathology, Peterborough District Hospital, Thorpe Road, Peterborough PE3 6DA). Briefly, the tissue was de-waxed in xylene, gradually re-hydrated through alcohol and washed in phosphate buffered saline (PBS) before being permeablised in 0.01 % Triton X-100 for 3 minutes followed by treatment with Proteinase K for 30 minutes at 37°C.

Direct *in situ* RT PCR was carried out in a GeneAmp In Situ PCR System 1000 (Perkin Elmer Biosystems) using a GeneAmp Thermostable rTth RT PCR kit (Perkin Elmer Biosystems). The primers used to amplify DTD were as follows:
Sense: 5'-gctggagtttatcaggtagcga-3' (SEQ ID NO: 7)
Antisense: 5'-ttgcatctacagccacactagg-3' (SEQ ID NO: 8)

The thermal cycling parameters were; 1 cycle of 94°C for 2.5 minutes followed by 20 cycles of 94°C for 40 seconds, 60°C for 50 seconds, and 72°C for 30 seconds. Amplified product was detectable through the direct incorporation of alkali stable Digoxigenin-11-dUTP (Roche Diagnostics Ltd.) which was added to the reaction mix. After washing in PBS an anti-Digoxigenin-Gold antibody (Roche Diagnostics Ltd.) was incubated on the tissue section for 30 minutes at room temperature, this was followed by a silver enhancement step (Roche Diagnostics Ltd. silver enhancement reagents) during which time the amplified expression product became visible by light microscopy. The tissue was counter-stained with hematoxylin (Dako Ltd.) and images were captured by a digital camera attached to a light microscope (x10 objective).

In the paired images of Figure 6 the upper panel demonstrates DTD expression in the breast cancer tissue, the lower panel represents a negative control consecutive section where the DTD primers have been replaced with non-specific primers. It is clearly apparent from these Figures that DTD is specifically expressed in the cancerous ductal epithelial cells of this breast cancer tissue (compare with surrounding breast tissue and negative control experiment). For example, a portion of epithelial hyperplasia (typical of breast carcinoma) has been flanked with two arrowheads (upper panel); this shows that the area of dark staining (representing BCMP101 1 expression) is restricted to the cancer cells (Figure 5).

In summary, DTD shows a restricted pattern of mRNA expression in normal human tissues, but is elevated in some breast cancer derived cell lines and multiple breast tumour tissues, suggesting that this protein has potential as a therapeutic target.

### Example 4: DTD Protein Expression in the MDA-MB-435 Breast Tumour Derived Cell Line

To examine DTD protein expression anti-DTD single chain Fv (ScFv) antibodies were generated. Briefly, DTD-derived peptide pools were conjugated to beads and panned with phage displayed ScFv's to isolate a small number of high affinity anti-DTD binders. The Western blot in Figure 6A illustrates specific binding of two of the ScFv's isolated (1 and 2). In this experiment binding of both ScFv's is inhibited by pre-incubation with the pooled peptides used for phage selection.

Figure 6B shows a Western blot of endogenous DTD protein expression in multiple cell lines using the anti-DTD ScFv's. Of the cell lines tested, highest DTD protein expression was identified in the MDA-MB-435 breast tumour cell line and to a lesser level in the PC3 prostate tumour cell line. This data is entirely consistent with the results of Figure 3 where the MDA-MB-435 cell line had highest mRNA expression levels.

## Claims

1. A method of screening for and/or diagnosis of breast cancer in a subject, which method comprises the step of detecting and/or quantifying in a biological sample obtained from said subject:
(i) a DTD polypeptide which
a) comprises or consists of the amino acid sequence shown in Figure 1 (SEQ ID NO: 1);
b) is a derivative having one or more amino acid substitutions, deletions or insertions relative to the amino acid sequence shown in Figure 1 (SEQ ID NO: 1) which retains the activity of DTD; or
c) is a fragment of a polypeptide as defined in a) or b) above, which is at least ten amino acids long which retains the activity of DTD;
wherein said step comprises the use of an antibody that specifically binds to the DTD polypeptide.

2. The method according to claim 1, wherein the level of said DTD polypeptide in a biological sample is compared with a predetermined reference range or control.

3. The method according to claim 1 or 2, wherein the antibody is immobilised on a solid phase.

4. The method according to claim 1, wherein the step of detecting comprises use of a labelled binding partner to said antibody or wherein said antibody is labelled with a detectable marker.

5. The method according to claim 1, wherein the antibody is monoclonal, polyclonal, chimeric, humanised or bispecific, or an epitope-binding fragment of any one thereof.

6. The use of an antibody which binds specifically to a DTD polypeptide as defined in claim 1 ,for the preparation of a medicament for the prophylaxis and/or treatment of breast cancer.

7. The use according to claim 6, wherein the antibody is monoclonal, polyclonal, chimeric, humanised or bispecific, or an epitope-binding fragment of any one thereof.

8. The use according to claim 6 or 7, wherein the antibody is conjugated to a therapeutic agent or a drug moiety.

9. The use according to claim 8, wherein the drug moiety is a toxin.

## Patentansprüche

1. Verfahren zum Screening auf und/oder zur Diagnose von Brustkrebs in einem Individuum, wobei man in einem Verfahrensschritt in einer von dem Individuum erhaltenen biologischen Probe folgendes nachweist und/oder quantifiziert:
(i) ein DTD-Polypeptid, das
a) die in Figur 1 (SEQ ID NO: 1) dargestellte Aminosäuresequenz umfaßt oder aus dieser besteht;
b) ein Derivat mit einer oder mehreren Aminosäuresubstitutionen, -deletionen oder -insertionen relativ zur in Figur 1 (SEQ ID NO: 1) dargestellten Aminosäuresequenz ist, bei dem die DTD-Aktivität erhalten ist; oder
c) ein Fragment eines Polypeptids mit der in a) oder b) oben angegebenen Bedeutung ist, das eine Länge von mindestens 10 Aminosäuren aufweist und bei dem die DTD-Aktivität erhalten ist;
wobei der genannte Schritt die Verwendung eines spezifisch an das DTD-Polypeptid bindenden Antikörpers umfaßt.

2. Verfahren nach Anspruch 1, wobei der Spiegel des DTD-Polypeptids in einer biologischen Probe mit einem vorbestimmten Referenzbereich oder einer Kontrolle verglichen wird.

3. Verfahren nach Anspruch 1 oder 2, wobei der Antikörper an einer Festphase immobilisiert ist.

4. Verfahren nach Anspruch 1, wobei der Nachweisschritt die Verwendung eines markierten Bindungspartners für den Antikörper umfaßt oder wobei der Antikörper mit einem nachweisbaren Marker markiert ist.

5. Verfahren nach Anspruch 1, wobei es sich bei dem Antikörper um einen monoklonalen, polyklonalen, chimären, humanisierten oder bispezifischen Antikörper oder ein epitopbindendes Fragment eines dieser Antikörper handelt.

6. Verwendung eines Antikörpers, der spezifisch an ein DTD-Polypeptid mit der in Anspruch 1 angegebenen Bedeutung bindet, zur Herstellung eines Arzneimittels zur Vorbeugung und/oder Behandlung von Brustkrebs.

7. Verwendung nach Anspruch 6, wobei es sich bei dem Antikörper um einen monoklonalen, polyklonalen, chimären, humanisierten oder bispezifischen Antikörper oder ein epitopbindendes Fragment eines dieser Antikörper handelt.

8. Verwendung nach Anspruch 6 oder 7, wobei der Antikörper an ein Therapeutikum oder eine Arzneistoffgruppierung konjugiert ist.

9. Verwendung nach Anspruch 8, wobei es sich bei der Arzneistoffgruppierung um ein Toxin handelt.

## Revendications

1. Méthode de dépistage et/ou de diagnostic du cancer du sein chez un sujet, ladite méthode comprenant l'étape consistant à détecter et/ou à quantifier dans un échantillon biologique obtenu dudit sujet :
(i) un polypeptide DTD qui
a) comprend ou est constitué de la séquence d'acides aminés présentée en figure 1 (SEQ ID NO : 1);
b) est un dérivé ayant une ou plusieurs substitutions, délétions ou insertions d'acides aminés par rapport à la séquence d'acides aminés présentée en figure 1 (SEQ ID NO : 1) qui conserve l'activité de DTD ; ou
c) est un fragment d'un polypeptide tel que défini en a) ou b) ci-dessus, qui fait au moins dix acides aminés de long et qui conserve l'activité de DTD ;
ladite étape comprenant l'utilisation d'un anticorps qui se lie spécifiquement au polypeptide DTD.

2. Méthode selon la revendication 1, selon laquelle le taux dudit polypeptide DTD dans un échantillon biologique est comparé à une gamme de référence prédéterminée ou à un témoin.

3. Méthode selon la revendication 1 ou 2, selon laquelle l'anticorps est immobilisé sur une phase solide.

4. Méthode selon la revendication 1, selon laquelle l'étape de détection comprend l'utilisation d'un partenaire de liaison marqué dudit anticorps ou selon laquelle ledit anticorps est marqué avec un marqueur détectable.

5. Méthode selon la revendication 1, selon laquelle l'anticorps est monoclonal, polyclonal, chimère, humanisé ou bispécifique, ou un fragment de l'un quelconque de ceux-ci se liant à un épitope.

6. Utilisation d'un anticorps qui se lie spécifiquement à un polypeptide DTD tel que défini dans la revendication 1, pour la préparation d'un médicament destiné à la prophylaxie et/ou au traitement du cancer du sein.

7. Utilisation selon la revendication 6, selon laquelle l'anticorps est monoclonal, polyclonal, chimère, humanisé ou bispécifique, ou un fragment de l'un quelconque de ceux-ci se liant à un épitope.

8. Utilisation selon la revendication 6 ou 7, selon laquelle l'anticorps est conjugué à un agent thérapeutique ou à un fragment de médicament.

9. Utilisation selon la revendication 8, selon laquelle le fragment de médicament est une toxine.
